(19) Europäisches Patentamt
European Patent Office
Office européen des brevets

(11) **EP 4 685 228 A1**

(12) **EUROPEAN PATENT APPLICATION**
published in accordance with Art. 153(4) EPC

(43) Date of publication:
**28.01.2026 Bulletin 2026/05**

(21) Application number: **23938625.3**

(22) Date of filing: **17.11.2023**

(51) International Patent Classification (IPC):
**C12N 5/077** (2010.01)    **A23L 13/00** (2016.01)

(52) Cooperative Patent Classification (CPC):
**A23L 13/00; C12N 5/06**

(86) International application number:
**PCT/KR2023/018525**

(87) International publication number:
**WO 2024/242255 (28.11.2024 Gazette 2024/48)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **23.05.2023  KR 20230066428**
**13.11.2023  KR 20230156641**

(71) Applicant: **Industry Academic Cooperation Foundation of Yeungnam University**
**Gyeongsan-si, Gyeongsangbuk-do 38541 (KR)**

(72) Inventors:
• CHOI, Inho
  Gyeongsan-si, Gyeongsangbuk-do 38688 (KR)
• LEE, Eun Ju
  Daegu 42511 (KR)
• LIM, Jeong Ho
  Gyeongsan-si, Gyeongsangbuk-do 38657 (KR)
• AHMAD, Syed Sayeed
  Gyeongsan-si, Gyeongsangbuk-do 38543 (KR)
• CHUN, Hee Jin
  Gyeongsan-si, Gyeongsangbuk-do 38449 (KR)

(74) Representative: **Roos, Peter**
**c/o Roospatent**
**Noldinstrasse 7**
**81545 München (DE)**

(54) **MEDIUM COMPOSITION FOR PROMOTING MUSCLE CELL DIFFERENTIATION COMPRISING QUERCETIN AS ACTIVE INGREDIENT**

(57)    The present invention relates to a medium composition for promoting muscle cell differentiation, the medium composition comprising quercetin as an active ingredient. Quercetin was found to promote the differentiation of muscle cells through the expression of muscle differentiation-related factors and the promotion of creatine kinase activity and can thus be effectively used as a composition for muscle cell differentiation or a medium composition for producing cultured meat.

[FIG.6]

**Description**

Technical Field

The present disclosure relates to a medium composition for promoting muscle cell differentiation, including quercetin as an active ingredient.

Background Art

According to the Food and Agriculture Organization of the United Nations (FAO), with the continues increase in the world's population, meat consumption is expected to increase by 1.3% annually from 3.04 million tons in 2018 to 4.55 million tons in 2050, with an announcement that more than 200 million tons of meat must be additionally produced each year to meet human demand for meat consumption. Food shortages due to future population growth bear various issues, including environmental and ethical concerns, and thus the development of alternative meats to address each of these issues is necessary. 'Alternative livestock products' that can replace these livestock products generally require less resource input and greenhouse gas consumption than existing meat and reduce generation of environmental pollutants such as bad odors.

Typical alternative meats that are receiving attention include plant-based alternative meats, edible insects, and cultured meats, among which "cultured meat" refers to edible meat that is grown through cell proliferation in a laboratory without directly raising and slaughtering livestock. While various methods are being proposed for the technology of manufacturing and processing cultured meat, the cultured meat is basically made by processes of differentiating animal muscle cells and culturing them in three-dimensional tissues, with processing followed.

Serum that is essential for culturing animal cells for cultured meat production is used by isolating from animals such as calves, horses, sheep, pigs, dogs, and goats, among which fetal bovine serum (FBS) is the most commonly used. However, the process of separating serum has the disadvantages of being unethical, environmentally unfriendly, and expensive. Accordingly, serum-free medium has recently been attracting attention as a substitute for fetal bovine serum, which is a cell culture medium that involves the use of serum, which has unethical, environmentally unfriendly, and high-cost issues while the use of serum is eliminated by producing various hormones and growth factors essential for cell culture by recombinant protein synthesis technology and adding them to a synthetic medium. However, high cost still matters since there is a limitation in the types of cell lines to be cultured in serum-free media, with the cumbersome process for cell line adaptation for culture, and the addition of recombinant proteins is required instead of using fetal bovine serum. Therefore, research is actively underway to produce cultured meat faster at lower cost than existing methods.

Disclosure of the Invention

Technical Goals

An object of the present disclosure is to provide a medium composition for promoting muscle cell differentiation, including quercetin as an active ingredient.

Another object of the present disclosure is to provide a medium composition for producing cultured meat, including quercetin as an active ingredient.

Another object of the present disclosure is to provide a method of producing cultured meat, including treating a medium with quercetin.

Technical Solutions

To achieve the above objects, the present disclosure provides a medium composition for promoting muscle cell differentiation, including quercetin as an active ingredient, wherein the quercetin promotes muscle cell differentiation by regulating expression of one or more selected from the group consisting of myoblast determination protein 1 (MYOD), myogenin, and myosin heavy chain.

In addition, the present disclosure provides a medium composition for producing cultured meat, including quercetin as an active ingredient, wherein the quercetin promotes cultured meat production by regulating expression of one or more selected from the group consisting of myoblast determination protein 1 (MYOD), myogenin, and myosin heavy chain.

In addition, the present disclosure provides a method of producing cultured meat, including treating muscle cells with quercetin, wherein the quercetin promotes cultured meat production by regulating expression of one or more selected from the group consisting of myoblast determination protein 1 (MYOD), myogenin, and myosin heavy chain.

Advantageous Effects

According to the present disclosure, it has been determined that quercetin promotes differentiation of muscle cells by stimulating expression of muscle differentiation-related factors and creatine kinase activity and thus may be usefully utilized as a composition for promoting muscle cell differentiation or producing cultured meat.

Brief Description of Drawings

FIG. 1 shows a result of an in silico analysis conducted to identify the intermolecular binding between quercetin and myostatin.

FIG. 2 shows results of root-mean-square deviation (RMSD) (A) and root mean square fluctuation (RMSF) (B) analysis conducted to identify a binding stability of quercetin and myostatin. Red indicates myostatin, and green indicates a conjugate of myostatin and quercetin.

FIG. 3 shows results of observing muscle cells under a microscope to identify an effect of quercetin on muscle cell proliferation. A indicates a mouse myoblast, B indicates a bovine muscle stem cell, C indicates a porcine muscle stem cell, and D indicates a chicken muscle stem cell.

FIG. 4 shows results of MTS analysis conducted to identify an effect of quercetin on muscle cell proliferation. A indicates a mouse myoblast, B indicates a bovine muscle stem cell, C indicates a porcine muscle stem cell, and D indicates a chicken muscle stem cell.

FIG. 5 shows results of observing muscle cells under a microscope to identify an effect of quercetin on muscle cell differentiation. A indicates a mouse myoblast, B indicates a bovine muscle stem cell, C indicates a porcine muscle stem cell, and D indicates chicken muscle stem cell.

FIG. 6 shows results of analyzing a creatine kinase activity to identify an effect of quercetin on muscle cell differentiation. A indicates a mouse myoblast, B indicates a bovine muscle stem cell, C indicates a porcine muscle stem cell, and D indicates a chicken muscle stem cell. $*P \leq 0.05$, $**P \leq 0.01$, $***P \leq 0.001$.

FIG. 7 shows results of analyzing an effect of quercetin on gene and protein expression of muscle differentiation-related factors. A indicates a mouse myoblast, B indicates a bovine muscle stem cell, C indicates a porcine muscle stem cell, and D indicates a chicken muscle stem cell. $*P \leq 0.05$, $**P \leq 0.01$, $***P \leq 0.001$.

FIG. 8 shows results of analyzing a medium pH (A) and creatine kinase activity (B) following quercetin treatment into a mouse myoblast differentiation medium to identify an effect of quercetin on pH of a differentiation medium. $*P \leq 0.05$, $**P \leq 0.01$, $***P \leq 0.001$.

Best Mode for Carrying Out the Invention

Hereinafter, the present disclosure will be described in more detail.

The present disclosure provides a medium composition for promoting muscle cell differentiation, including quercetin as an active ingredient, wherein the quercetin promotes muscle cell differentiation by regulating expression of one or more selected from the group consisting of myoblast determination protein 1 (MYOD), myogenin, and myosin heavy chain.

The quercetin may promote creatine kinase activity and exhibit an antioxidant activity.

Additionally, the quercetin may be contained in an amount of 1 to 1000 nM.

The muscle cell may be derived from one or more animals selected from the group consisting of, but not limited to, chicken, cow, pig, and mouse.

The medium composition may further include a component for muscle cell growth and differentiation in addition to quercetin. The quercetin may be included in the medium composition and may be used alone as a culture medium supplement.

In addition, the present disclosure provides a medium composition for producing cultured meat, including quercetin as an active ingredient, wherein the quercetin promotes cultured meat production by regulating expression of one or more selected from the group consisting of myoblast determination protein 1 (MYOD), myogenin, and myosin heavy chain.

The cultured meat may be derived from muscle cells of one or more animals selected from the group consisting of chicken, cow, and pig, but is not limited thereto.

The term "cultured meat" as used herein refers to an alternative meat produced by proliferating cells taken from living animals, and it is mainly produced by culturing animal tissues using stem cells and has recently been gaining attention as a type of meat that is obtained without raising and slaughtering livestock. Cultured meat has the advantage of minimizing issues concerning animal welfare, ethics, and environment since it is based on tissue culture and eliminates the need for livestock farming. However, there are still disadvantages such as high production cost per kg and extended cultivation time.

In addition, the present disclosure provides a method of producing cultured meat, including treating muscle cells with quercetin, wherein the quercetin promotes cultured meat production by regulating expression of one or more selected from the group consisting of myoblast determination protein 1 (MYOD), myogenin, and myosin heavy chain.

Modes for Carrying Out the Invention

Hereinafter, the present disclosure will be described in more detail through examples to help understanding of the present disclosure. However, examples below are merely intended to illustrate the present disclosure, and the scope of the present disclosure is not limited to the following examples. Examples of the present disclosure are provided to more completely explain the present disclosure to those skilled in the art.

[Experimental Example 1] Preparation of muscle cells

Mouse myoblast cell line (C2C12) used was purchased from American Type Culture Collection (ATCC). Muscle stem cells were collected from leg muscles of cows, pigs, and chickens, minced, and reacted with 1% Pronase enzyme at 37°C for 2 hours, followed by centrifugation at 1,000 g for 3 minutes. After centrifugation, the supernatant was removed, and the tissue was suspended in Dulbecco's Modified Eagle's Medium (DMEM) supplemented with 1% penicillin/streptomycin (hereinafter referred to as P/S) and 10% fetal bovine serum (hereinafter referred to as FBS), and then the tissue and cells were separated through a 100 $\mu$m filter. The separated cells were centrifuged at 1,000 g for 5 minutes, the supernatant was removed, and the cells were suspended in Ham's F10 growth medium containing 1% P/S, 20% FBS, and 5 ng/mL fibroblast growth factor 2 (FGF2), after which cells were transferred to a cell culture dish and cultured at 37°C in the presence of 5% $CO_2$.

[Experimental Example 2] Analysis of proliferation and differentiation of muscle cells

2-1. Analysis of muscle cell proliferation

To identify the effect of quercetin on muscle stem cell proliferation, myoblasts and muscle stem cells being cultured in proliferation medium (Ham's F10 containing 1% P/S, 20% FBS, and 5 ng/mL FGF2) were treated with various concentrations of quercetin and cultured for 4 days. To compare proliferation rates, MTS analysis was performed. Myoblasts and muscle stem cells were cultured in a proliferation medium for 4 days, and after removing all of the medium, a culture medium in which proliferation medium and MTS reagent are mixed in a ratio of 8:2 was treated to the cells, followed by a reaction for 1 hour. The reacted culture medium was transferred to a 96-well microplate in a volume of 100 $\mu$L, and the absorbance of the culture medium contained in each well was measured at 490 nm using a microplate.

2-2. Analysis of muscle cell differentiation

To determine the effect of quercetin on muscle stem cell differentiation, myoblasts and muscle stem cells being differentiated were treated with different concentrations of quercetin in differentiation medium (Ham's F10 containing 1% P/S, 20% FBS, and 5 ng/mL FGF2) and cultured for 4 days. To compare differentiation rates, creatine kinase activity was analyzed. Myoblasts and muscle stem cells were cultured in differentiation medium for 4 days, then all media were removed, followed by washing with phosphate buffer saline (PBS), 200 $\mu$L of new saline was added, and the cells were scraped and collected. Afterwards, the cells were disrupted through sonication, and the supernatant was separated through centrifugation. For analysis of creatine kinase activity, a mixture (100 $\mu$L of buffer, 10 $\mu$L of substrate, and 1 $\mu$L of enzyme) was prepared, and 100 $\mu$L of the mixture was treated to 10 $\mu$L of the supernatant. Afterwards, the absorbance at 340 nm was measured at 20 and 25 minutes after the start of the reaction using a microplate reader, and the value of creatine kinase activity (U/L) was calculated using the following Equation 1.

*Creatine kinase activity (U/L) = (25-minute O.D value - 20-minute O.D value) / (Calibrator O.D value - Distilled water O.D value)* $\times$ *600*　　　　[Equation 1]

[Experimental Example 3] RNA extraction and cDNA synthesis

1 mL of TRIzol (TRIzol™) reagent was added to the cultured cells, and the cells were crushed. 200 $\mu$Lof chloroform was added to the crushed cells, and centrifugation was performed at 12,000 rpm for 10 minutes. After centrifugation, the upper clear water layer was transferred to a new tube, followed by addition of 500 $\mu$Lof isopropanol.

Afterwards, the mixture was mixed sufficiently and centrifuged at 12,000 rpm for 10 minutes. Thereafter, all supernatants except the RNA pellet were removed, and 1 mL of 70% ethanol was added, followed by washing. Afterwards, all ethanol was removed, and the RNA pellet was dissolved in distilled water containing diethylpyrocarbonate (hereinafter referred to as DEPC). The obtained RNA was quantified using a spectrophotometer, and then 2 μg of total RNA was reacted with reverse transcriptase to synthesize cDNA.

[Experimental Example 4] Analysis of expression of factors related to muscle differentiation

4-1. Analysis of gene expression

To identify an effect of quercetin on gene expression of muscle differentiation-related factors [myoblast determination protein 1 (hereinafter referred to as MYOD), myogenin (hereinafter referred to as MYOG), and myosin heavy chain (hereinafter referred to as MYH)], real-time PCR was performed. Synthesized cDNA and primers designed using NCBI's primer blast tool; and reagents containing SYBR green fluorescent substance were mixed. Real-time PCR was analyzed via the 7500 real-time PCR from Applied Biosystems. The sequences of the PCR primers are as shown in Table 1 below.

TABLE 1

| Primer | Size (bp) | Forward | Reverse |
|---|---|---|---|
| GAPDH | 157 | 5'-caacatcaaatgggcagatg-3'(SEQ. 1) | 5'-gacacccatcacaaacatgg-3'(SEQ.2) |
| MYOD | 160 | 5'-agctctcgcaggagaaacag-3'(SEQ.3) | 5'-ctggaggcagtatgggacat-3'(SEQ.4) |
| MYOG | 205 | 5'-cgccatccagtacatcgag-3'(SEQ.S) | 5'-atcgctcaggaggtgatctg-3'(SEQ.6) |
| MYH | 141 | 5'-ctgccgatgaaaaagtggcta-3'(SEQ.7) | 5'-agccttgtctgcaacttctg-3'(SEQ.8) |
| The temperature was the same for all at 59°C | | | |

4-2. Analysis of protein expression

To determine the effect of quercetin on the protein expression of muscle differentiation-related factors (MYOD, MYOG, and MYH), Western blot was performed. All media of cells being cultured were removed and washed with saline solution. A mixture containing RIPA buffer and 1% protease inhibitor was added to the cells and scraped. After centrifugation at 12,000 rpm for 10 minutes, the supernatant was recovered and quantified using a spectrophotometer. 60 μg of the extracted protein was electrophoresed on a 10% acrylamide gel and transferred to a polyvinylidene fluoride (PVDF) membrane (Milipore). Blocking was performed for 1 hour in tris-buffered saline (TBS) buffer containing 3% skim milk and 1% surfactant (tween-20), and the primary antibody was added. After reacting for 16 hours at 4°C, washing was performed three times for 10 minutes in TBS buffer containing 1% surfactant (Tween-20), followed by a reaction with the secondary antibody for 1 hour at room temperature. Afterwards, washing was performed three times for 10 minutes in TBS buffer containing 1% surfactant (tween-20), followed by development after adding Super Signal West Pico Chemiluminescent Substrate.

[Experimental Example 5] Statistical Analysis

Tukey's test was performed to analyze the differences between the means of gene expression. The software used was SAS version 9.0, and one-way ANOVA was conducted using PROC GLM. A value of $p \leq 0.05$ was considered to indicate statistical significance.

[Example 1] Analysis of binding between quercetin and myostatin

1-1. Analysis of intermolecular binding

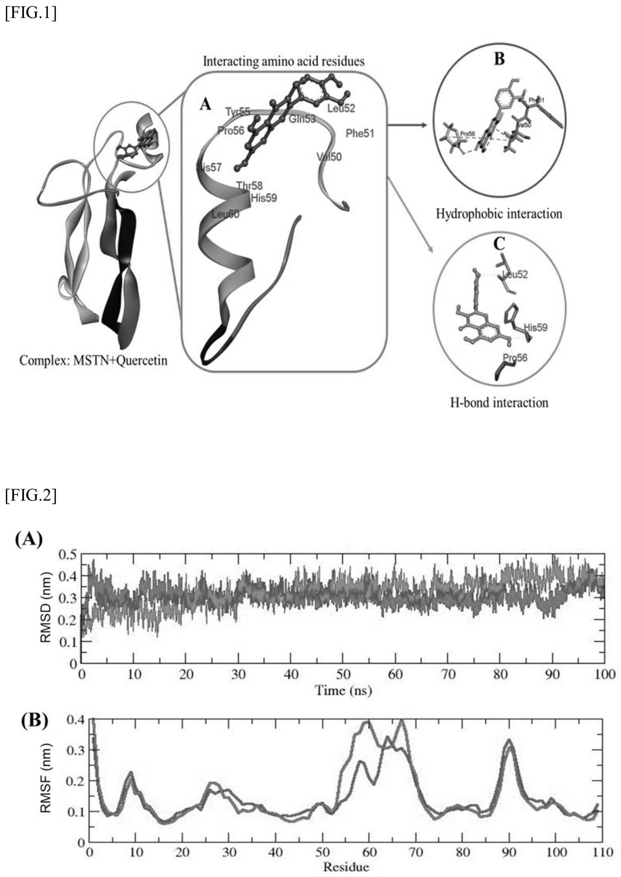

As a result of conducting in silico analysis, to identify the intermolecular binding between quercetin and myostatin, quercetin and myostatin were found to interact with a binding free energy of -7.40 kcal/mol, and quercetin was shown to interact with amino acid residues constituting myostatin (Val50, Phe51, Leu52, Gln53, Tyr55, Pro56, His57, Thr58, His59, and Leu60), as shown in FIG. 1.

1-2. Analysis of binding stability

As a result of conducting a molecular dynamics simulation study, to identify the binding stability of quercetin and myostatin, the root-mean-square deviation (RMSD) index showed that the complex of quercetin and myostatin (green) formed a more stable complex 10 ns after binding compared to myostatin (red) alone, as shown in FIG. 2A. Additionally, as shown in FIG. 2B, the root mean square fluctuation (RMSF) index showed a decrease at residues 50 to 70 of the complex of quercetin and myostatin (green) compared to myostatin alone (red). From the above results, it was revealed that the stability of amino acid residues in the complex further increased at residues 50 to 70.

[Example 2] Analysis of muscle cell proliferation and differentiation

2-1. Analysis of muscle cell proliferation

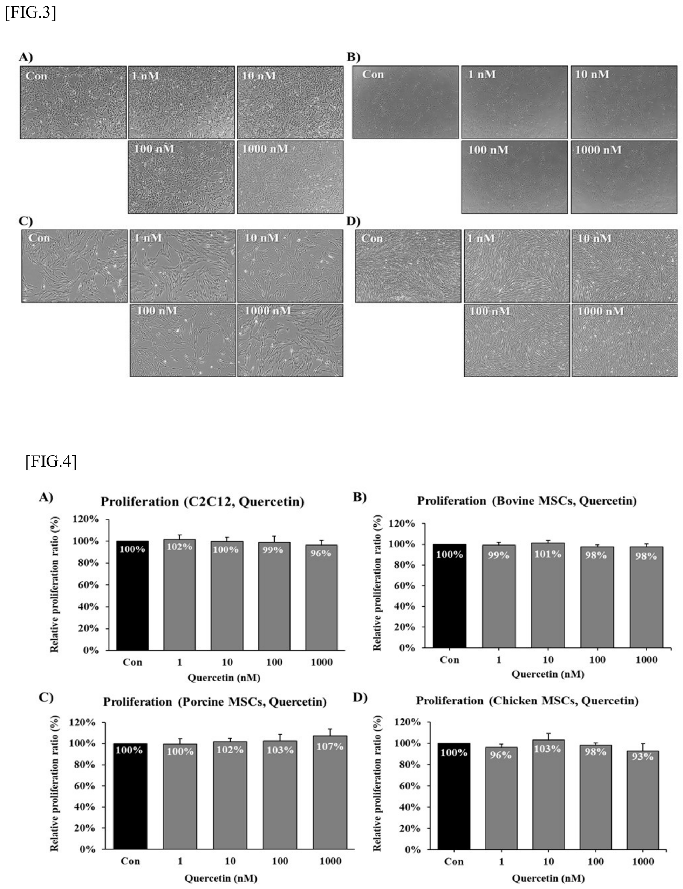

According to the Experimental Example 2, in order to identify the effect of quercetin on muscle cell proliferation, quercetin was added at various concentrations during the proliferation culture of myoblasts and muscle stem cells, and after culturing for 4 days, observation was made under a microscope, followed by MTS analysis for comparison of the proliferation rate. As a result, as shown in FIGS. 3 and 4, no significant change in proliferation rate was observed in any muscle cell depending on whether quercetin was treated or not.

2-2. Analysis of muscle cell differentiation

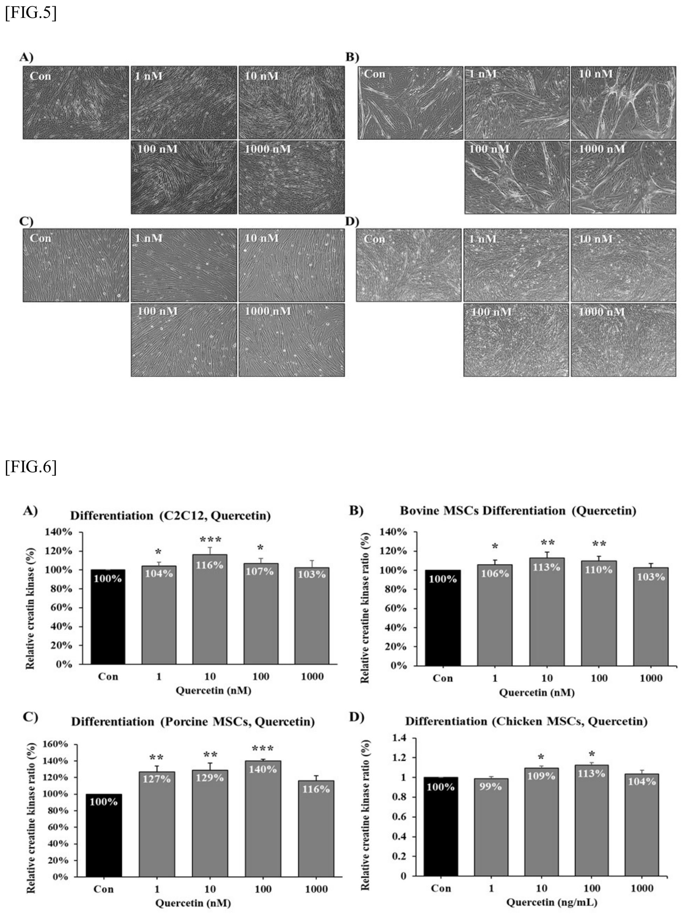

According to the Experimental Example 2, in order to identify the effect of quercetin on muscle cell differentiation, quercetin was added at various concentrations during differentiation culture of myoblasts and muscle stem cells, and after culturing for 4 days, observation was made under a microscope, followed by analysis of creatine kinase activity to compare the differentiation rate. As a result, as shown in FIG. 5, the diameter of myotubes of myoblasts and muscle stem cells increased in the 10 nM and 100 nM treatment groups, and as shown in FIG. 6, it was found that the creatine kinase activity of myoblasts and muscle stem cells significantly increased in 10 nM and 100 nM.

[Example 3] Analysis of expression of factors related to muscle differentiation

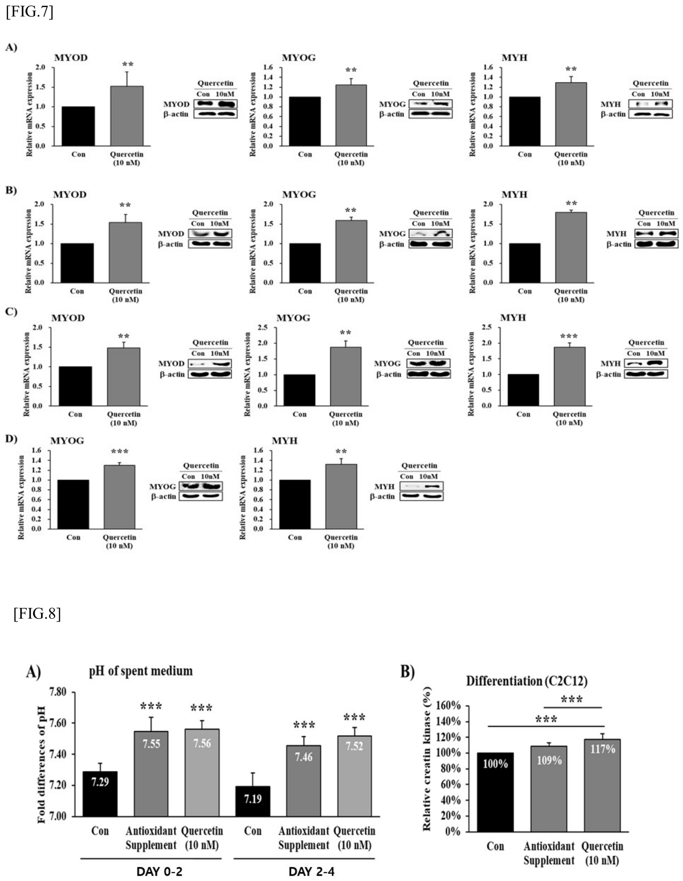

According to the above Experimental Example 4, to identify the effect of quercetin on the expression of factors related to muscle differentiation, 10 nM quercetin was added to the process of differentiating myoblasts and muscle stem cells, and gene and protein expression of the factors was analyzed. As a result, as shown in FIG. 7, MYOG and MYH expression significantly increased upon quercetin treatment in all muscle cells, and MYOD expression significantly increased upon quercetin treatment in mouse myoblasts and bovine and porcine muscle stem cells.

[Example 4] Analysis of the effect of quercetin on medium pH

To determine the effect of quercetin on medium pH, mouse myoblasts were cultured for 0 to 2 days in differentiation medium containing 10 nM quercetin, and after replacing the medium on the second day of differentiation, the cells were cultured for an additional 2 to 4 days, followed by pH measurement. As a positive control, a commercially available antioxidant supplement (1000x, Sigma-Aldrich, product number: A1345-5ML) was used after being diluted 1,000-fold according to the manufacturer's instructions. As a result, as shown in FIG. 8A, the pH in the quercetin treatment group increased compared to the control group (Con; untreated group), showing a pH value similar to that of the positive control group. In addition, as shown in FIG. 8B, as a result of analyzing the creatine kinase activity of the experimental group, the creatine kinase activity increased by 17% in the quercetin treatment group and 9% in the antioxidant supplement treatment group compared to the control group (Con). From the results, it was determined that quercetin accelerates muscle differentiation through antioxidant activity.

While a specific part of the present disclosure has been described in detail above, it is clear for those skilled in the art that this specific description is merely preferred example embodiments, and the scope of the present disclosure is not limited thereby. In other words, the substantial scope of the present disclosure is defined by the appended claims and their equivalents.

**Claims**

1. A medium composition for promoting muscle cell differentiation, the medium composition comprising quercetin as an active ingredient, wherein the quercetin promotes muscle cell differentiation by regulating expression of one or more selected from the group consisting of myoblast determination protein 1 (MYOD), myogenin, and myosin heavy chain.

2. The medium composition of claim 1, wherein the quercetin promotes creatine kinase activity.

3. The medium composition of claim 1, wherein the quercetin exhibits an antioxidant activity.

4. The medium composition of claim 1, wherein the quercetin is contained in an amount of 1 to 1000 nM.

5. The medium composition of claim 1, wherein the muscle cell is derived from one or more animals selected from the group consisting of chicken, cow, pig, and mouse.

6. A medium composition for producing cultured meat, the medium composition comprising quercetin as an active ingredient, wherein the quercetin promotes cultured meat production by regulating expression of one or more selected from the group consisting of myoblast determination protein 1 (MYOD), myogenin, and myosin heavy chain.

7. The medium composition of claim 6, wherein the cultured meat is derived from muscle cells of one or more animals selected from the group consisting of chicken, cow, and pig.

8. A method of producing cultured meat, comprising treating muscle cells with quercetin, wherein the quercetin promotes cultured meat production by regulating expression of one or more selected from the group consisting of myoblast determination protein 1 (MYOD), myogenin, and myosin heavy chain.

[FIG.1]

[FIG.2]

[FIG.3]

[FIG.4]

[FIG.5]

A)

B)

C)

D)

[FIG.6]

A) Differentiation (C2C12, Quercetin)

B) Bovine MSCs Differentiation (Quercetin)

C) Differentiation (Porcine MSCs, Quercetin)

D) Differentiation (Chicken MSCs, Quercetin)

[FIG.7]

[FIG.8]

## INTERNATIONAL SEARCH REPORT

<table>
<tr><td>International application No.</td></tr>
<tr><td>**PCT/KR2023/018525**</td></tr>
</table>

**A.    CLASSIFICATION OF SUBJECT MATTER**

**C12N 5/077**(2010.01)i; **A23L 13/00**(2016.01)i

According to International Patent Classification (IPC) or to both national classification and IPC

**B.    FIELDS SEARCHED**

Minimum documentation searched (classification system followed by classification symbols)

C12N 5/077(2010.01); A23L 13/00(2016.01); A61K 31/353(2006.01); A61K 31/7048(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 퀘르세틴(quercetin), 근육 세포(muscle cell), 배양육(cultured meat), 미오디 (MYOD, myoblast determination protein 1), 미오게닌(myogenin), 미오신 중쇄(myosin heavy chain)

**C.    DOCUMENTS CONSIDERED TO BE RELEVANT**

| Category* | Citation of document, with indication, where appropriate, of the relevant passages | Relevant to claim No. |
|---|---|---|
| X | CN 114958730 A (NANJING AGRICULTURAL UNIVERSITY) 30 August 2022 (2022-08-30)<br>See abstract; paragraph [0047]; example 3; and claims 10-23. | 1-8 |
| X | HOUR, T.-C. et al. The promotion of migration and myogenic differentiation in skeletal muscle cells by quercetin and underlying mechanisms. Nutrients. 2022, vol. 14, thesis no.:4106, pp. 1-19.<br>See abstract; figure 10; and pages 2-3, 13 and 15. | 1-8 |
| A | ZHU, C. et al. Effect of quercetin on muscle growth and antioxidant status of the dark sleeper Odontobutis potamophila. Frontiers in Genetics. 2022, vol. 13, thesis no.:938526, pp. 1-15.<br>See entire document. | 1-8 |
| A | US 2018-0104269 A1 (SUNTORY HOLDINGS LIMITED) 19 April 2018 (2018-04-19)<br>See entire document. | 1-8 |
| A | KR 10-2022-0011301 A (CJ CHEILJEDANG CORPORATION) 28 January 2022 (2022-01-28)<br>See entire document. | 1-8 |

☐ Further documents are listed in the continuation of Box C.          ☑ See patent family annex.

| | |
|---|---|
| *    Special categories of cited documents:<br>"A"  document defining the general state of the art which is not considered to be of particular relevance<br>"D"  document cited by the applicant in the international application<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| Date of the actual completion of the international search | Date of mailing of the international search report |
|---|---|
| **14 March 2024** | **15 March 2024** |

| Name and mailing address of the ISA/KR | Authorized officer |
|---|---|
| **Korean Intellectual Property Office<br>Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT
### Information on patent family members

International application No.

**PCT/KR2023/018525**

| Patent document cited in search report | | | Publication date (day/month/year) | Patent family member(s) | | | Publication date (day/month/year) |
|---|---|---|---|---|---|---|---|
| CN | 114958730 | A | 30 August 2022 | None | | | |
| US | 2018-0104269 | A1 | 19 April 2018 | CA | 2982645 | A1 | 03 November 2016 |
| | | | | CN | 107530320 | A | 02 January 2018 |
| | | | | HK | 1246182 | A1 | 07 September 2018 |
| | | | | JP | 7013238 | B2 | 31 January 2022 |
| | | | | SG | 10201909412 | A | 28 November 2019 |
| | | | | SG | 11201708158 | A | 29 November 2017 |
| | | | | TW | 201713330 | A | 16 April 2017 |
| | | | | WO | 2016-175136 | A1 | 03 November 2016 |
| KR | 10-2022-0011301 | A | 28 January 2022 | KR | 10-2023-0079340 | A | 07 June 2023 |

Form PCT/ISA/210 (patent family annex) (July 2022)